# EUROPEAN PATENT APPLICATION

(11) **EP 4 230 123 A1**
(43) Date of publication of application: **23.08.2023**
(21) Application number: 23155651.5
(22) Date of filing: 08.02.2023
(51) Int. Cl.: A61B 5/02

(54) **OPTICAL SENSOR DEVICES FOR HEMORRHAGE DETECTION**

(30) Priority: 18.02.2022 CN 202210150086
(71) Applicant: Honeywell International Inc., Charlotte, NC 28202 (US)
(72) Inventor: HOU, Zhixiong, Charlotte, 28202 (US); LUO, Rui, Charlotte, 28202 (US); ZHAO, Linan, Charlotte, 28202 (US); KENDALL, Matthew, Charlotte, 28202 (US); WANG, Yuyan, Charlotte, 28202 (US); HUANG, Chuang, Charlotte, 28202 (US)
(74) Representative: Haseltine Lake Kempner LLP

(57) **Abstract**

Devices, apparatuses, methods, and computer program products are provided for hemorrhage detection. An example sensing device for hemorrhage detection includes a blood detection layer that includes a permeable film and one or more optical fibers supported by the permeable film. The device also includes an optical sensor that includes a light source that emits light and an optical receiver in optical communication with the light source via the one or more optical fibers. The optical receiver generates optical data responsive to the light emitted by the light source and received by the optical receiver via the one or more optical fibers indicative of at least a wavelength of the light. The device further includes a controller operably coupled with the optical sensor that receives the optical data generated by the optical receiver in response to the emitted light and determines an amount of blood based upon the optical data.

## Description

### TECHNOLOGICAL FIELD

Example embodiments of the present disclosure relate generally to medical applications and, more particularly, to devices and methods for improved hemorrhage detection that leverage optical sensors.

### BACKGROUND

Hospitals, clinics, and other medical institutions or environments care for a variety of patients dealing with an array of differing conditions. As part of caring for patients, these institutions may rely upon various diagnostic systems, sensors, and/or the like to provide up-to-date data associated with the current condition of one or more patients. The inventors have identified numerous deficiencies with these existing technologies in the field of medical monitoring devices, the remedies for which are the subject of the embodiments described herein.

### BRIEF SUMMARY

Devices, methods, systems, and associated computer program products are provided for hemorrhage detection. An example sensing device for hemorrhage detection may include a blood detection layer including a permeable film and one or more optical fibers supported by the permeable film, an optical sensor, and a controller. The optical sensor may include a light source configured to emit light and an optical receiver in optical communication with the light source via the one or more optical fibers. The optical receiver may be configured to generate optical data responsive to the light emitted by the light source and received by the optical receiver via the one or more optical fibers where the optical data is indicative of at least a wavelength of the light. The controller may be operably coupled with the optical sensor and configured to receive the optical data generated by the optical receiver in response to the light emitted by the light source and determine an amount of blood received by the blood detection layer based upon the optical data.

In some embodiments, the controller may be further configured to compare the optical data with an alert threshold and generate an alert signal in an instance in which the optical data satisfies the alert threshold.

In some further embodiments, the alert signal may be configured to cause presentation of a user notification.

In other further embodiments, the alert signal is configured to modify an operating condition of one or more systems communicably coupled with the controller.

In some embodiments, the one or more optical fibers may be supported by a first surface of the permeable film.

In some further embodiments, the sensing device may further include an anti-penetration layer applied to a second surface of the permeable film, wherein the second surface is opposite the first surface.

In some embodiments, the anti-penetration layer may include a polyethylene (PE) or polypropylene (PP) microporous film configured to emit vapor to an external environment of the sensing device but preclude fluid transmission therethrough.

In some further embodiments, the sensing device may further include a first diversion layer defining a first surface and a second surface opposite the first surface, wherein the second surface of the first diversion layer is applied to the first surface of the blood detection layer.

In some embodiments, the first diversion layer may include an air through non-woven of polyethylene (PE) and polyethylene terephthalate (PET) bicomponent fibers or PE and polypropylene (PP) bicomponent fibers.

In some further embodiments, the sensing device may further include an absorption layer defining a first surface and a second surface opposite the first surface, wherein the second surface of the absorption layer is applied to the first surface of the first diversion layer.

In some embodiments, the absorption layer may include a superabsorbent polymer (SAP). In such an embodiment, one or more bicomponent fibers of the first diversion layer may be arranged along a lengthwise direction of the first diversion layer.

In some further embodiments, the sensing device may include a second diversion layer defining a first surface and a second surface opposite the first surface, wherein the second surface of the second diversion layer is applied to the first surface of the absorption layer.

In some embodiments, the second diversion layer may include an air through nonwoven of polyethylene (PE) and polyethylene terephthalate (PET) bicomponent fibers or PE and polypropylene (PP) bicomponent fibers.

In some further embodiments, the sensing device may include a hydrophobic layer defining a first surface and a second surface opposite the first surface, wherein the second surface of the hydrophobic layer is applied to the first surface of the second diversion layer.

In some embodiments, the hydrophobic layer may include a spunbound nonwoven material or an air through non-woven polyethylene (PE) or polypropylene (PP) material.

The above summary is provided merely for purposes of summarizing some example embodiments to provide a basic understanding of some aspects of the disclosure. Accordingly, it will be appreciated that the above-described embodiments are merely examples and should not be construed to narrow the scope or spirit of the disclosure in any way. It will be appreciated that the scope of the disclosure encompasses many potential embodiments in addition to those here summarized, some of which will be further described below.

### BRIEF DESCRIPTION OF THE DRAWINGS

Having described certain example embodiments of the present disclosure in general terms above, reference will now be made to the accompanying drawings. The components illustrated in the figures may or may not be present in certain embodiments described herein. Some embodiments may include fewer (or more) components than those shown in the figures.
FIG. 1 illustrates an example sensing device for hemorrhage detection of the present disclosure in accordance with some example embodiments described herein;
FIGS. 2A-2B illustrate side and top views, respectively, of an example blood detection layer and optical sensor of the sensing device of FIG. 1 in accordance with some example embodiments described herein;
FIG. 3 illustrates an example hydrophobic layer for use with example sensing devices described herein;
FIG. 4 illustrates an example second diversion layer for use with example sensing devices described herein;
FIG. 5 illustrates an example absorption layer for use with example sensing devices described herein;
FIG. 6 illustrates an example first diversion layer for use with example sensing devices described herein;
FIG. 7 illustrates an example anti-penetration layer for use with example sensing devices described herein;
FIG. 8 illustrates a schematic block diagram of an example controller that may perform various operations in accordance with some example embodiments described herein;
FIG. 9 illustrates an example flowchart for hemorrhage detection in accordance with some example embodiments described herein; and
FIG. 10 illustrates an example flowchart for alert signal operations in accordance with some example embodiments described herein.

### DETAILED DESCRIPTION

Some embodiments of the present disclosure will now be described more fully hereinafter with reference to the accompanying drawings, in which some, but not all embodiments of the disclosure are shown. Indeed, this disclosure may be embodied in many different forms and should not be construed as limited to the embodiments set forth herein; rather, these embodiments are provided so that this disclosure will satisfy applicable legal requirements. Like numbers refer to like elements throughout. As used herein, terms such as "front," "rear," "top," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

As used herein, the phrases "in one embodiment," "according to one embodiment," "in some embodiments," and the like generally refer to the fact that the particular feature, structure, or characteristic following the phrase may be included in at least one embodiment of the present disclosure. Thus, the particular feature, structure, or characteristic may be included in more than one embodiment of the present disclosure such that these phrases do not necessarily refer to the same embodiment.

As used herein, the word "example" is used herein to mean "serving as an example, instance, or illustration." Any implementation described herein as "example" is not necessarily to be construed as preferred or advantageous over other implementations.

As used herein, the terms "data," "content," "information," "electronic information," "signal," "command," and similar terms may be used interchangeably to refer to data capable of being transmitted, received, and/or stored in accordance with embodiments of the present disclosure. Thus, use of any such terms should not be taken to limit the spirit or scope of embodiments of the present disclosure. Further, where a first device is described herein to receive data from a second device, it will be appreciated that the data may be received directly from the second device or may be received indirectly via one or more intermediary computing devices, such as, for example, one or more servers, relays, routers, network access points, base stations, hosts, and/or the like, sometimes referred to herein as a "network." Similarly, where a first device is described herein as sending data to a second device, it will be appreciated that the data may be sent directly to the second device or may be sent indirectly via one or more intermediary computing devices, such as, for example, one or more servers, remote servers, cloud-based servers (e.g., cloud utilities), relays, routers, network access points, base stations, hosts, and/or the like.

As used herein, the term "computer-readable medium" refers to non-transitory storage hardware, non-transitory storage device or non-transitory computer system memory that may be accessed by a computing device, a microcomputing device, a computational system or a module of a computational system to encode thereon computer-executable instructions or software programs. A non-transitory "computer-readable medium" may be accessed by a computational system or a module of a computational system to retrieve and/or execute the computer-executable instructions or software programs encoded on the medium. Exemplary non-transitory computer-readable media may include, but are not limited to, one or more types of hardware memory, non-transitory tangible media (for example, one or more magnetic storage disks, one or more optical disks, one or more USB flash drives), computer system memory or random access memory (such as, DRAM, SRAM, EDO RAM), and the like.

As described herein, the sensing device embodiments of the present disclosure reference an example sensing device for hemorrhage detection (e.g., detecting the presence or an amount of blood received by the described sensing device). The present disclosure, however, contemplates that the embodiments of the present disclosure may also be configured to determine any characteristic associated with blood such that the described "amount" may refer to a volume, density, and/or the presence of blood received by the sensing device (e.g., the detection of an amount of blood may refer to any event including but not limited to a hemorrhage of an associated patient). Additionally, the described "amount" may refer to a speed or volume change and/or a time required for the volume of blood received by the blood detection layer or the sensing device to satisfy an associated threshold. Furthermore, although described hereinafter with reference to detection of blood received by the sensing device, the present disclosure contemplates that the sensing devices described herein may be equally applicable to detection of other fluids, compounds, elements, etc. without limitation. For example, the sensing devices described herein may, additionally or alternatively, generate optical data that is associated with light having a wavelength indicative of another fluid (e.g., any fluid other the blood embodiments described herein) so as to determine an amount of such other fluid (e.g., sweat, tissue fluid, urine, etc.) received by the sensing device.

### Overview

As described above, hospitals, clinics, and other medical institutions or environments care for a variety of patients dealing with an array of differing conditions, and these institutions may rely upon various diagnostic systems, sensors, and/or the like to provide up-to-date data associated with the current condition of one or more patients. In many instances, however, traditional systems for detecting a bleeding related event (e.g., a hemorrhage or the like) rely upon ad hoc methods where individual caretakers must determine the condition of a patient. Said differently, these traditional systems fail to provide a data-driven, standardized method and device for detecting bleeding related events resulting in increased risk to patients. In many instances, such as with high risk patients, pregnant patients, and/or the like, failure to timely detect hemorrhaging of the patient may result in critical consequences. By way of a particular example, cesarean section (C-section) procedures are often associated with potential risk for bleeding events that may be dangerous to both the mother and child. As described above, traditional attempts, if any, at detecting a bleeding event associated with a C-section procedure fail to provide timely, data-driven determinations that reliably reduce the risk associated with these procedures.

Accordingly, the sensing devices, systems, methods, and computer program products of the present disclosure provide a mechanism for reliably detecting bleeding related events (e.g., hemorrhaging or the like) by leveraging blood detection layers with optical sensors. For example, embodiments of the present disclosure may, via a controller or other computing device, generate optical data of light passing through optical fiber(s) of a blood detection layer that is indicative of the wavelength of this light. The controller may further determine the presence of blood (e.g., the occurrence of a hemorrhage) and/or an amount of blood received by the blood detection layer based upon this optical data (e.g., detected wavelength) and generate an alert signal that notifies a user (e.g., caretaker, physician, etc.) of the bleeding event (e.g., hemorrhage or otherwise). In some embodiments, the sensing device may further form a multi-layer surgery accessory that operates to (1) ensure appropriate blood distribution for effective contact with the blood detection layer and optical fibers supported thereon, (2) improve comfort for associated patients, and/or (3) improve cleanliness via a disposable configuration.

### Sensing Device

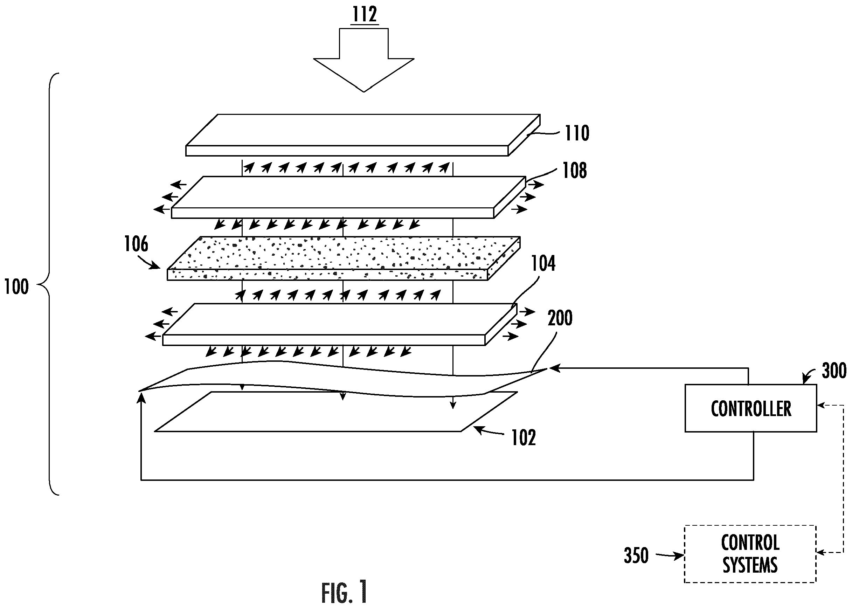

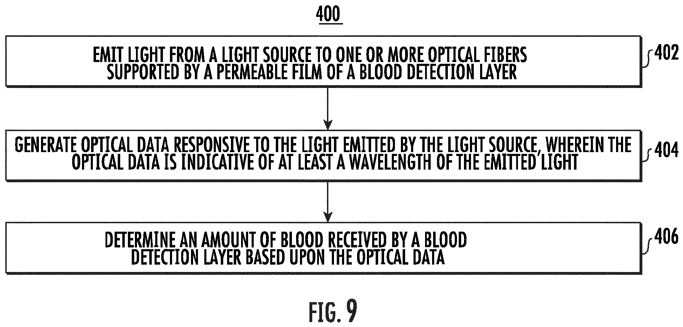

With reference to FIG. 1, an example sensing device 100 (e.g., device 100) of the present disclosure is illustrated. As shown, the sensing device 100 may include a blood detection layer 200 that is operably coupled with a controller 300. As described hereafter with reference to the operations of FIGS. 9-10, the controller 300 may be configured to cause light to be emitted by a light source through the blood detection layer 200 (e.g., propagated by optical fiber(s) of the blood detection layer 200) and, in response to the blood received by the blood detection layer 200, determine optical data (e.g., at least a wavelength of the light transmitted through optical fibers of the blood detection layer 200) indicative of an amount of blood received by the blood detection layer 200. In some embodiments, the sensing device 100 may include only the blood detection layer 200, optical sensor, and the controller 300. In other embodiments, as shown in FIG. 1, the sensing device 100 may be formed as a multi-layer functional fabric that comprises many different layers as described hereafter. As such, the present application contemplates that the sensing device 100 may include any number of layers in addition to the blood detection layer 200 based upon the intended application of the device 100.

As detailed hereafter, the sensing device 100 of the present disclosure may be used in medical applications, such as in conjunction with a C-section procedure, in that at least a portion of the sensing device 100 may be disposed proximate a patient. For example, at least a portion of the sensing device 100 (e.g., the blood detection layer 200) may be position proximate an incision, wound, stitches, sutures, etc. of a patient such that any bleeding of said incision, wound, stitches, sutures, etc. may be directed to or otherwise contact the blood detection layer 200 of the sensing device 100. As such, the blood detection layer 200 may be dimensioned (e.g., sized and shaped) so as to be positioned relative the body of a patient. The total amount of fluid that may be absorbed by the sensing device 100 (e.g., by the various materials described herein) may similarly vary based upon the intended application of the device 100. For example, the thickness and/or density of the sensing device 100 may be determined so as to absorb a defined amount of fluid (e.g., 500 mL, 1,000 mL, 1500 mL, and/or the like).

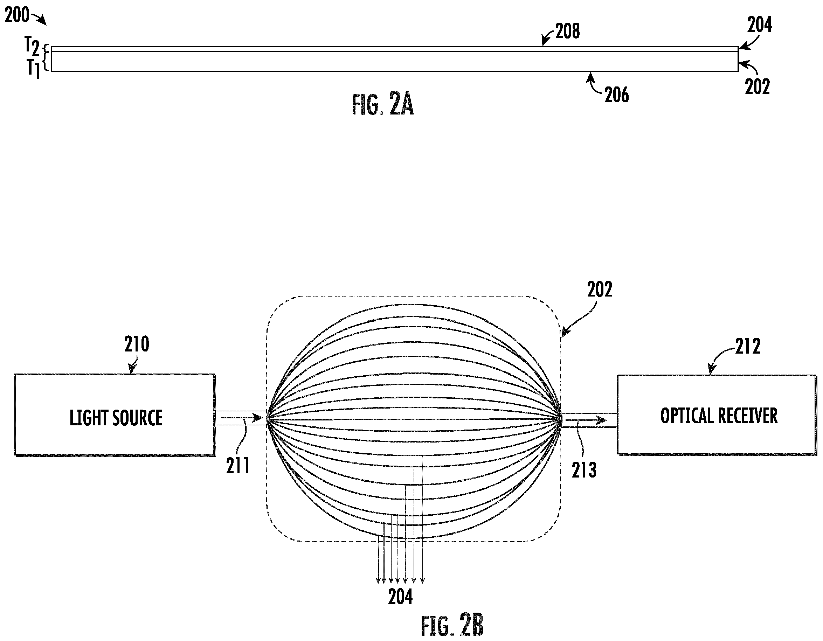

With reference to FIGS. 2A-2B, the blood detection layer 200 may define or otherwise be formed of a permeable film 202. The permeable film 202 may, for example be formed of or otherwise comprise a polyurethane film through which fluid (e.g., blood) may pass. In some non-limiting embodiments, for example, the permeable film 202 may have a thickness T₁ of approximately 100 µm. The permeable film 202 may further define a first surface 208 and a second surface 206 opposite the first surface 208 such that a body having the thickness Ti extends therebetween. Although described herein with reference to a permeable polyurethane material, the present disclosure contemplates that the permeable film 202 of the blood detection layer 200 may be formed of or comprise any permeable membrane or film based upon the intended application of the sensing device 100.

The blood detection layer 200 may further include one or more optical fibers 204 supported by the permeable film 200. The one or more optical fibers 204 may comprise a medium through which optical signals (e.g., light) may propagate. By way of example, the one or more optical fibers 204 may comprise one or more strands, fibers, or the like formed of glass through which light 211 emitted by a light source 210 may travel. Although described herein with reference to optical fibers 204, the present disclosure contemplates that the blood detection layer 200 may include any optical communication medium of any material through which light may propagate based upon the intended application of the sensing device 100. Furthermore, the present disclosure contemplates that the particular size, orientation, shape, dimensions, number, etc. of the one or more optical fibers 204 may vary based upon the intended application of the sensing device 100. Although described herein with reference to one or more optical fibers 204 supported on the permeable film 202, the present disclosure contemplates that "supporting" may equally refer to instances in which the one or more optical fibers 204 are at least partially embedded in the first surface 208 of the permeable film 202.

The sensing device 100 may further include an optical sensor that includes a light source 210 and an optical receiver 212. The light source 210 may be optically coupled with an end of the one or more optical fibers 204 disposed proximate the light source 210 such that light 211 emitted by the light source 210 may be received by the one or more optical fibers 204. The present disclosure contemplates that the light source 210 may refer to any device capable of generating optical signals or light, such as a laser (e.g., vertical cavity surface emitting laser (VCSEL)), light-emitting diode (LED), lamp, bulb, etc. As would be evident in light of the optical data generation operations described hereinafter, the light 211 emitted by the light source may be white light (e.g., light having a wavelength between approximately 400 - 700 nm) that is received by the one or more optical fibers 204 so as to propagate therethrough. Although described herein with reference to light 211 having a wavelength associated with white light in the visual spectrum, the present disclosure contemplates that light having any number of wavelengths in the visual spectrum may be used based upon the intended application of the sensing device 100. In some embodiments, the light 211 emitted by the light source 210 may have any wavelength other than a wavelength associated with the red colored light in the visual spectrum (e.g., other than a wavelength of approximately 620-750 nm).

The optical sensor may further include an optical receiver 212 (e.g., photodiode (PD)) or the like optically coupled with an opposing end of the one or more optical fibers 204 so as to be in optical communication with the light source 210. By way of example, the optical receiver 212 may comprise a photodiode that is configured to receive the light 211 emitted by the light source 210 and transmitted via the one or more optical fibers 204. The optical receiver 212 may be configured to generate optical data responsive to the light emitted 211 by the light source 210 and received by the optical receiver 212 via the one or more optical fibers 204. As described further with reference to the operations of FIGS. 9-10, the optical data may be indicative of at least a wavelength of the light received by the optical receiver 212. The optical receiver 212 may comprise circuitry components, processors, photoelectric sensor components, wavelength sensor components, or the like configured to generate optical data that is indicative of the wavelength of the light 213 that exits the one or more optical fibers 204 and is received by the optical receiver 212. In other words, the light 211 that is emitted by the light source 210 may have a wavelength that differs from the wavelength of the same light 213 exiting the one or more optical fibers 204. Said differently, the light emitted by the light source 210 when viewed by the optical receiver 212 after the light has propagated through the one or more optical fibers 204 may have a wavelength that is influenced by fluid (e.g., blood 112) received by the blood detection layer 200.

By way of a particular example, a patient's blood (e.g., blood 112 in FIG. 1) may include one or more erythrocytes (e.g., red blood cells) that may be red in color (e.g., have a wavelength associated with red visible light). The optical fibers 204 may contact this blood 112 when blood 112 is received by the blood detection layer 200 such that the blood 112 may impact or otherwise influence the wavelength of the light 211 emitted by the light source 210, such that the wavelength of the light 213 that is received by the optical receiver 212 differs from the wavelength of the light 211 emitted by the light source 210 as described above. The optical receiver 212 may be configured to detect the wavelength of the light 213 received by the optical receiver 212 so as to generate optical data that is indicative of at least this wavelength. By way of example, the optical receiver 212 may include, define, or otherwise comprise a light transmittance detector that is sensitive to red light (e.g., light in the visible spectrum having a wavelength indicative of the color red). By way of a more particular example, the optical receiver 212 may be configured to detect the presence of light 213 having a wavelength of approximately 620-750 nm (e.g., a wavelength associated with red light). As such, the controller 300 described hereafter may receive this optical data that is indicative of the wavelength of the light 213 received by the optical receiver 212 (e.g., the wavelength of the light propagating through the one or more optical fibers 204 as impacted by the blood 112) and detect the presence and/or amount of blood received by the blood detection layer 200.

In addition to this detector configured for identifying the presence of red visible light, the present disclosure contemplates that the blood detection layer 200 and optical sensor (e.g., light source 210 and optical receiver 212) may be configured so as to only be influenced by wavelengths associated with red visible light. For example, the sensing device 100 may, in operation, contact various other fluids (e.g., tissue fluid, urine, saline solutions, amniotic fluid, water, etc.) due to its relative position to a patient. These fluids, however, do not have a wavelength associated with red visible light (i.e., these fluids have a wavelength other than approximately 620-750 nm). In doing so, the embodiments described herein may be configured to accurately determine the presence and/or amount of blood while ignoring the presence of other fluids. By way of example, the wavelength of red light (e.g., approximately 620-750 nm) may have a linear relationship with the concentration of blood received by the blood detection layer 200. As such, a relatively small amount of blood (e.g., drops of blood or the like) may result in a relatively small impact (e.g., based on this linear relationship) on the wavelength of the light received by the optical receiver 212. The optical data generated by the optical receiver 212 may be reflective of this relatively small impact (e.g., a lower percentage wavelength transmittance). As described hereafter, when sufficient blood is present, the wavelength of red light transmittance increases (e.g., a particular percentage increase over a defined time period) as indicative of a hemorrhaging event or the like.

As described hereafter with reference to a determination of an amount of blood received by the blood detection layer 200, the controller 300 may perform one or more calibration procedures in which various known amounts of blood are provided to blood detection layers (e.g., such as blood detection layer 200) with corresponding wavelengths determined by the optical receiver (e.g., generated optical data). With a known amount (e.g., volume, density, etc.) of blood supplied to the sensing device 100, the optical receiver 212 may generate optical data indicative of the wavelength of the light propagating though the one or more optical fibers 204, and the controller 300 may be calibrated, via leveraging one or more statistical methods, regressions, etc. In other embodiments, the controller 300 may be operably connected with one or more control systems 350 that may, for example, provide calibrated data for use in forthcoming blood amount determinations by the controller 300. By way of example, the control systems 350 may, as described hereafter, be associated with one or more diagnostic systems, drug delivery systems, or the like for a particular patient and, as such, may include patient data indicative of the particular wavelength of the patient's blood.

As described above, in some embodiments, the sensing device 100 may only include the blood detection layer 200 and the controller 300 operably connected thereto. In such an embodiment, the blood detection layer 200 may be housed within an exterior liner, enclosure, or the like (not shown) to prevent damage to the optical sensor (e.g., light source 210 and/or optical receiver 212), one or more optical fibers 204, etc. In such an embodiment, the controller 300, light source 210, and/or optical receiver 212 may be commonly housed within said exterior liner, enclosure, or the like (not shown) in order to provide an integrated solution. In other embodiments, the controller 300, light source 210, optical receiver 212, etc. may be housed separate from the blood detection layer 200. For example, the controller 300 may be removably attached to the blood detection layer 200 so as to provide a disposable solution (e.g., disposing of the blood detection layer 200 following use). In other embodiments, the controller 300 may be communicably and/or operably coupled with the blood detection layer 200 via a network as described hereafter.

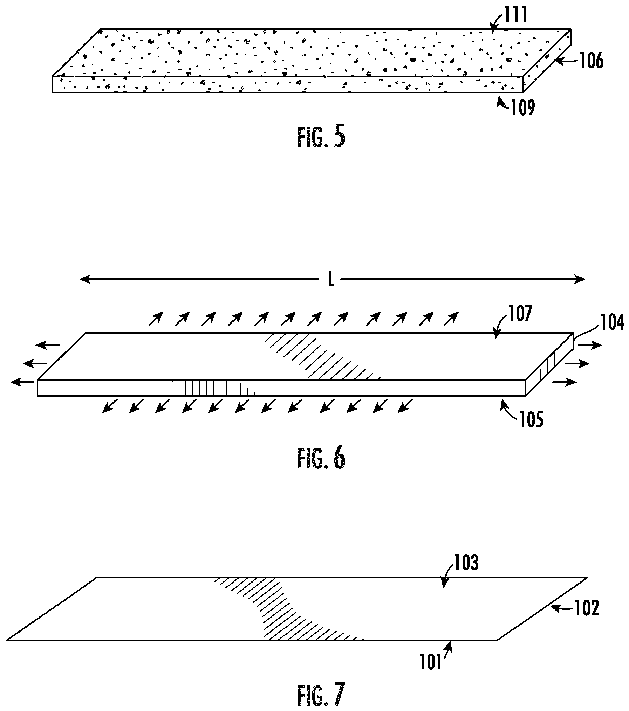

In some embodiments, as shown in FIG. 1, the sensing device 100 may further include one or more other layers combined with the blood detection layer 200. For example, as shown in FIG. 1 and FIG. 7, the sensing device 100 may further include an anti-penetration layer 102 applied to a second surface 206 of the permeable film 202. The anti-penetration layer 102 as shown in FIG. 1 may be configured to be downstream of the blood detection layer 200 in that blood (e.g., blood 112) leaving the blood detection layer 200 is received by the anti-penetration layer 102. The anti-penetration layer 102 may be formed of or otherwise comprise a polyethylene (PE) or polypropylene (PP) microporous film configured to emit vapor, via one or more micropores defined by the anti-penetration layer 102, to an external environment of the sensing device 100 but preclude fluid transmission therethrough. Said differently, the anti-penetration layer 102 may be configured to increase the comfort to the patient while preventing contamination. For example, fluid vapor may be emitted by the microporous holes defined by the anti-penetration layer 102 from the first surface 103 to an external environment of the sensing device 100 proximate the second surface 101 of the anti-penetration layer 102. Fluid, however, may not pass therethrough so as to prevent contamination (e.g., patient blood contacting unintended surfaces, people, etc.) while improving cleanliness (e.g., a disposable solution).

The anti-penetration layer 102 may be dimensioned (e.g., sized and shaped) based upon the corresponding dimensions of the blood detection layer 200. For example, the anti-penetration layer 102 may be the same size or larger than the blood detection layer 200 such that blood (e.g., blood 112) may be received by the anti-penetration layer 102 from the blood detection layer 200. The anti-penetration layer 102 may be applied to the second surface 206 of the blood detection layer 200 and attached thereto, such as via an adhesive, hot melt procedure, or the like. Although described herein with reference to an adhesive, the present disclosure contemplates that any other mechanism for attaching or affixing the anti-penetration layer 102 with the blood detection layer 200 may be used.

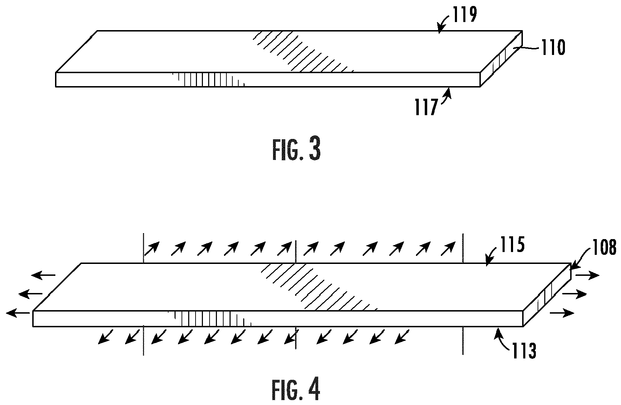

With reference to FIGS. 1 and 4, the sensing device 100 may also include a first diversion layer 104 defining a first surface 107 and a second surface 105 opposite the first surface 107. The second surface 105 of the first diversion layer 104 may be applied to the first surface 208 of the blood detection layer 200. In some embodiments, the first diversion layer 104 may be formed of or otherwise comprise an air through non-woven of polyethylene (PE) and polyethylene terephthalate (PET) bicomponent fibers or PE and polypropylene (PP) bicomponent fibers. As would be evident in light of the present disclosure, in order to accurately determine the wavelength of the light as impacted by the blood received by the blood detection layer 200, the blood 112 may substantially evenly distributed, disbursed, or diverted along the surface area of the blood detection layer 200. In order to achieve this uniform or substantially uniform distribution (e.g., to prevent blood concentration at a particular location of the blood detection layer 200), the first diversion layer 104 may be configured to slow the flow of the blood 112. In some embodiments, the structure of the first diversion layer 104 may further facilitate the distribution of the blood 112 by defining microchannels or the like that promote fluid distribution via capillary action. The first diversion layer 104 may be dimensioned (e.g., sized and shaped) based upon the corresponding dimensions of the blood detection layer 200. In some embodiments, one or more bicomponent fibers of the first diversion layer 104 may be arranged along a lengthwise direction of the first diversion layer 104 in order to improve the blood distribution operations described above. The first diversion layer 104 may be applied to the first surface 208 of the blood detection layer 200 and attached thereto, such as via an adhesive, hot melt procedure, or the like. Although described herein with reference to an adhesive, the present disclosure contemplates that any other mechanism for attaching or affixing the first diversion layer 104 with the blood detection layer 200 may be used.

The sensing device 100 may also include an absorption layer 106 defining a first surface 111 and a second surface 109 opposite the first surface 111. The second surface 109 of the absorption layer 106 may be applied to the first surface 107 of the first diversion layer 104. In some embodiments, the absorption layer 106 may be formed of or otherwise comprise a superabsorbent polymer (SAP), such as fluff pulp with sodium polyacrylate or the like. In order to control the volume of blood 112 that ultimately reaches the blood detection layer 200, the absorption layer 106 may operate to absorb the blood 112 at a defined rate (e.g., based upon the SAP material selection or the like) such that a substantially consistent volume (e.g., a volume known to the controller 300) of blood may pass through the absorption layer 106. The absorption layer 106 may be dimensioned (e.g., sized and shaped) based upon the corresponding dimensions of the blood detection layer 200 or any other layer described herein. The absorption layer 106 may be applied to the first surface 107 of the first diversion layer 104 and attached thereto, such as via an adhesive, hot melt procedure, or the like. Although described herein with reference to an adhesive, the present disclosure contemplates that any other mechanism for attaching or affixing the absorption layer 106 with the first diversion layer 104 may be used. The absorption layer 106 may comprise or be formed of a fluff pulp with an SAP material, viscus nonwovens, cotton nonwovens, and/or a combination of the above. The SAP material may include a superabsorbent resin of macromolecules containing hydrophilic groups with a crosslinked structure including one or more of a starch (e.g., graft, carboxymethylation, etc.), cellulose (e.g., carboxymethylation, graft, etc.), synthetic polymer (e.g., polyacrylic acid, polyvinyl alcohol, polyoxyethylene, etc.), among others.

With reference to FIGS. 1 and 3, the sensing device 100 may also include a second diversion layer 108 defining a first surface 115 and a second surface 113 opposite the first surface 115. The second surface 113 of the second diversion layer 108 may be applied to the first surface 111 of the absorption layer 106. In some embodiments, the second diversion layer 108 may also be formed of or otherwise comprise an air through non-woven of polyethylene (PE) and polyethylene terephthalate (PET) bicomponent fibers or PE and polypropylene (PP) bicomponent fibers. Similar to the first diversion layer 104, the second diversion layer 108 may operate to substantially evenly distribute, disburse, or divert blood 112 along the surface area of one or more layers (e.g., the absorption layer 106) of the sensing device 100. In order to achieve this uniform or substantially uniform distribution (e.g., prevent blood concentration at a particular location of the absorption layer 106), the second diversion layer 108 may be configured to slow the flow of the blood 112. In some embodiments, the structure of the second diversion layer 108 may also facilitate the distribution of the blood 112 by defining microchannels or the like that promote fluid distribution via capillary action. The second diversion layer 108 may be dimensioned (e.g., sized and shaped) based upon the corresponding dimensions of the blood detection layer 200 or any other layer described herein. In some embodiments, one or more bicomponent fibers of the second diversion layer 108 may also be arranged along a lengthwise direction of the second diversion layer 108 in order to improve the blood distribution operations described above. The second diversion layer 108 may be applied to the first surface 111 of the absorption layer 106 and attached thereto, such as via an adhesive, hot melt procedure, or the like. Although described herein with reference to an adhesive, the present disclosure contemplates that any other mechanism for attaching or affixing the second diversion layer 108 with the absorption layer 106 may be used.

With continued reference to FIGS. 1 and 3, the sensing device 100 may further include a hydrophobic layer 110 defining a first surface 119 and a second surface 117 opposite the first surface 119. The second surface 117 of the hydrophobic layer 110 may be applied to the first surface 115 of the second diversion layer 108. In some embodiments, the hydrophobic layer 110 may be formed of or otherwise comprise a spunbond nonwoven material or an air through non-woven polyethylene (PE) or polypropylene (PP) material. As described above, the sensing device 100 described herein may be positioned proximate an incision, wound, stitches, sutures, etc. of a patient such that any bleeding of said incision, wound, stitches, sutures, etc. may be directed to or otherwise contact the blood detection layer 200 of the sensing device 100. In some embodiments, the hydrophobic layer 110 may serve as the exterior layer of the sensing device 100 and, as such, may be dimensioned (e.g., sized and shaped) so as to be positioned relative the body of a patient. The hydrophobic nature of this layer 110 may be configured to increase the comfort of a patient in contact with the hydrophobic layer 110 by providing substantially dry contact between the sensing device 100 and the patient. In some embodiments, the hydrophobic layer 110 may serve as the exterior layer and/or housing of one or more of the layers described herein of the sensing device 100 (e.g., so as to at least partially enclose one or more of the layers described herein). Although described herein with reference to an air through non-woven polyethylene (PE) or polypropylene (PP) material, the hydrophobic layer 110 may be formed of or otherwise comprise nylon 6 (P6), nylon 66 (Pa66), Terryl^{™}(Pa56), and/or the like. Furthermore, the hydrophobic layer 110 is not limited to woven, knitting, and/or nonwoven fabrics.

### Example Computing Device and Control Systems

As shown in FIG. 1, the sensing device 100 may include a controller 300 that is operably or communicably coupled with blood detection layer 200, the light source 210, and/or the optical receiver 212 (e.g., the optical sensor), and, in some embodiments, one or more control systems 350. In some instances, the sensing device 100 may comprise or otherwise support the controller 300, the light source 210, and/or the optical receiver 212, in whole or in part, such that the sensing device 100 is formed as a single, integrated device. In other embodiments, the controller 300 may be operably coupled with the blood detection layer 200, light source 210, optical receiver 212, and/or sensing device 100 via a detachable/pluggable wired connection (not shown) or a network (not shown).

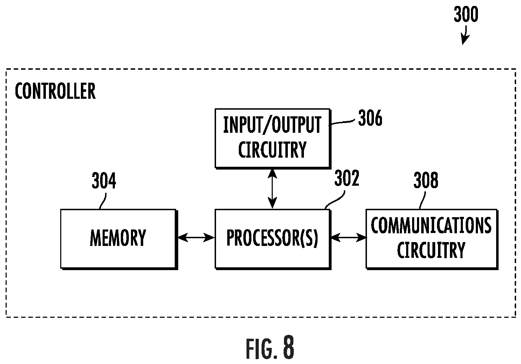

The controller 300 may include circuitry, networked processors, or the like configured to perform some or all of the apparatus-based processes described herein and may be any suitable processing device and/or network server. In this regard, the controller 300 may be embodied by any of a variety of devices. For example, the controller 300 may be configured to receive/transmit data (e.g., optical data, alert signal data, etc.) and may include any of a variety of fixed terminals, such as a server, desktop, or kiosk, or it may comprise any of a variety of mobile terminals, such as a portable digital assistant (PDA), mobile telephone, smartphone, laptop computer, tablet computer, or in some embodiments, a peripheral device that connects to one or more fixed or mobile terminals. Example embodiments contemplated herein may have various form factors and designs but will nevertheless include at least the components illustrated in FIG. 8 and described in connection therewith. The controller 300 may, in some embodiments, comprise several servers or computing devices performing interconnected and/or distributed functions. Despite the many arrangements contemplated herein, the controller 300 is shown and described herein as a single computing device to avoid unnecessarily overcomplicating the disclosure.

As described above, in some instances, the controller 300 may be operably coupled with the blood detection layer 200, light source 210, optical receiver 212, and/or the control systems 350 via a network. By way of example, the controller 300 may be associated with a central management system or central computing device configured to, in whole or in part, transmit instructions to or control operation of a plurality of devices. In such an embodiment, the network may include one or more wired and/or wireless communication networks including, for example, a wired or wireless local area network (LAN), personal area network (PAN), metropolitan area network (MAN), wide area network (WAN), or the like, as well as any hardware, software and/or firmware for implementing the one or more networks (e.g., network routers, switches, hubs, etc.). For example, the network may include a cellular telephone, mobile broadband, long term evolution (LTE), GSM/EDGE, UMTS/HSPA, IEEE 802.11, IEEE 802.16, IEEE 802.20, Wi-Fi, dial-up, and/or WiMAX network. Furthermore, the network may include a public network, such as the Internet, a private network, such as an intranet, or combinations thereof, and may utilize a variety of networking protocols now available or later developed including, but not limited to TCP/IP based networking protocols. In some embodiments, the network may refer to a collection of wired connections such that blood detection layer 200, the light source 210, the optical receiver 212, the control systems 350, and/or the controller 300 may be physically connected, via one or more networking cables or the like.

As illustrated in FIG. 8, the controller 300 may include a processor 302, a memory 304, input/output circuitry 306, and communications circuitry 308. The controller 300 may be configured to execute the operations described below in connection with FIGS. 9-10. Although components 302-308 are described in some cases using functional language, it should be understood that the particular implementations necessarily include the use of particular hardware. It should also be understood that certain of these components 302-308 may include similar or common hardware. For example, two sets of circuitry may both leverage use of the same processor 302, memory 304, communications circuitry 308, or the like to perform their associated functions, such that duplicate hardware is not required for each set of circuitry. The use of the term "circuitry" as used herein includes particular hardware configured to perform the functions associated with respective circuitry described herein. As described in the example above, in some embodiments, various elements or components of the circuitry of the controller 300 may be housed within components of the sensing device 100. It will be understood in this regard that some of the components described in connection with the controller 300 may be housed within one or more of the devices of FIGS. 1-7, while other components are housed within another of these devices, or by yet another device not expressly illustrated in FIGS. 1-7.

Of course, while the term "circuitry" should be understood broadly to include hardware, in some embodiments, the term "circuitry" may also include software for configuring the hardware. For example, although "circuitry" may include processing circuitry, storage media, network interfaces, input/output devices, and the like, other elements of the controller 300 may provide or supplement the functionality of particular circuitry.

In some embodiments, the processor 302 (and/or co-processor or any other processing circuitry assisting or otherwise associated with the processor) may be in communication with the memory 304 via a bus for passing information among components of the controller 300. The memory 304 may be non-transitory and may include, for example, one or more volatile and/or non-volatile memories. In other words, for example, the memory may be an electronic storage device (e.g., a non-transitory computer readable storage medium). The memory 304 may be configured to store information, data, content, applications, instructions, or the like, for enabling the controller 300 to carry out various functions in accordance with example embodiments of the present disclosure.

The processor 302 may be embodied in a number of different ways and may, for example, include one or more processing devices configured to perform independently. Additionally or alternatively, the processor may include one or more processors configured in tandem via a bus to enable independent execution of instructions, pipelining, and/or multithreading. The use of the term "processing circuitry" may be understood to include a single core processor, a multi-core processor, multiple processors internal to the computing device, and/or remote or "cloud" processors.

In an example embodiment, the processor 302 may be configured to execute instructions stored in the memory 304 or otherwise accessible to the processor 302. Alternatively or additionally, the processor 302 may be configured to execute hard-coded functionality. As such, whether configured by hardware or by a combination of hardware with software, the processor 302 may represent an entity (e.g., physically embodied in circuitry) capable of performing operations according to an embodiment of the present disclosure while configured accordingly. Alternatively, as another example, when the processor 302 is embodied as an executor of software instructions, the instructions may specifically configure the processor 302 to perform the algorithms and/or operations described herein when the instructions are executed.

The controller 300 further includes input/output circuitry 306 that may, in turn, be in communication with processor 302 to provide output to a user and to receive input from a user, user device, or another source. In this regard, the input/output circuitry 306 may comprise a display that may be manipulated by a mobile application. In some embodiments, the input/output circuitry 306 may also include additional functionality including a keyboard, a mouse, a joystick, a touch screen, touch areas, soft keys, a microphone, a speaker, or other input/output mechanisms. The processor 302 and/or user interface circuitry comprising the processor 302 may be configured to control one or more functions of a display through computer program instructions (e.g., software and/or firmware) stored on a memory accessible to the processor (e.g., memory 304, and/or the like), such as to display a user notification as described herein.

The communications circuitry 308 may be any means such as a device or circuitry embodied in either hardware or a combination of hardware and software that is configured to receive and/or transmit data from/to a network and/or any other device, circuitry, or module in communication with the controller 300. In this regard, the communications circuitry 308 may include, for example, a network interface for enabling communications with a wired or wireless communication network. For example, the communications circuitry 308 may include one or more network interface cards, antennae, buses, switches, routers, modems, and supporting hardware and/or software, or any other device suitable for enabling communications via a network. Additionally or alternatively, the communication interface may include the circuitry for interacting with the antenna(s) to cause transmission of signals via the antenna(s) or to handle receipt of signals received via the antenna(s). These signals may be transmitted by the controller 300 using any of a number of wireless personal area network (PAN) technologies, such as Bluetooth' v1.0 through v3.0, Bluetooth Low Energy (BLE), infrared wireless (e.g., IrDA), ultra-wideband (UWB), induction wireless transmission, or the like. In addition, it should be understood that these signals may be transmitted using Wi-Fi, Near Field Communications (NFC), Worldwide Interoperability for Microwave Access (WiMAX) or other proximity-based communications protocols.

In addition, computer program instructions and/or other type of code may be loaded onto a computer, processor or other programmable circuitry to produce a machine, such that the computer, processor other programmable circuitry that execute the code on the machine create the means for implementing the various functions, including those described in connection with the components of controller 300.

As described above and as will be appreciated based on this disclosure, embodiments of the present disclosure may be configured as apparatuses, systems, methods, and the like. Accordingly, embodiments may comprise various means including entirely of hardware or any combination of software with hardware. Furthermore, embodiments may take the form of a computer program product comprising instructions stored on at least one non-transitory computer-readable storage medium (e.g., computer software stored on a hardware device). Any suitable computer-readable storage medium may be utilized including non-transitory hard disks, CD-ROMs, flash memory, optical storage devices, or magnetic storage devices.

In some embodiments, the sensing device 100 and/or the controller 300 may be operably or communicably coupled with one or more control systems 350. By way of example with reference to a hospital or medical implementation, the sensing device 100 of the present disclosure may integrate or otherwise communicate with various diagnostic systems, sensors, and/or the like (e.g., one or more control systems 350) to provide up-to-date data associated with the current condition of one or more patients. By way of a particular example, the sensing device 100 and/or controller 300 may be operably coupled with a warning or alert system, one or more drug delivery systems, or the like and may further be configured to modify or otherwise control operation thereof. As such, the one or more control systems 350 may similarly comprise circuitry components, such as those described with reference to the controller 300, in order to perform their respective operations.

### Example Operations

FIG. 9 illustrates a flowchart containing a series of operations for hemorrhage detection (e.g., method 400). The operations illustrated in FIG. 9 may, for example, be performed by, with the assistance of, and/or under the control of an apparatus (e.g., controller 300), as described above. In this regard, performance of the operations may invoke one or more of processor 302, memory 304, input/output circuitry 306, and/or communications circuitry 308.

As shown in operation 402, the apparatus (e.g., controller 300) includes means, such as processor 302, communications circuitry 308, or the like, for emitting light from a light source to one or more optical fibers supported by a permeable film of a blood detection layer. As described above, the light source may be optically coupled with an end of the one or more optical fibers disposed proximate the light source such that light emitted by the light source may be received by the one or more optical fibers. The controller 300 may, continuously, periodically, according to a determined sampling rate, and/or the like, transmit instructions to the light source so as to cause the light source to emit light. As described above, the light emitted by the light source may be white light (e.g., light having a wavelength between approximately 400 - 700 nm) and may be received by the one or more optical fibers so as to propagate therethrough. Although described herein with reference to light having a wavelength associated with white light in the visual spectrum, the present disclosure contemplates the controller 300 may cause light to be emitted by the light source having any number of wavelengths in the visual spectrum m based upon the intended application of the sensing device 100. In some embodiments, the controller 300 may cause light to be emitted by the light source having any wavelength other than a wavelength associated with the color red in the visual spectrum (e.g., other than a wavelength of approximately 620-750 nm).

As shown in operation 404, the apparatus (e.g., controller 300) includes means, such as processor 302, communications circuitry 308, or the like, for generating, by an optical receiver in optical communication with the light source via the one or more optical fibers, optical data responsive to the light emitted by the light source. The optical data generated by the optical receiver may be indicative of at least a wavelength of the emitted light. As described above, the optical receiver (e.g., photodiode (PD) or the like) optically coupled with an opposing end of the one or more optical fibers may be be in optical communication with the light source so as to receive the light emitted by the light source and transmitted via the one or more optical fibers. The optical receiver may comprise circuitry components, processors, photoelectric sensor components, wavelength sensor components, or the like configured to generate optical data that is indicative of the wavelength of the light that exits the one or more optical fibers and is received by the optical receiver. In other words, the light that is emitted by the light source may have a wavelength that differs from the wavelength of the same light exiting the one or more optical fibers, such that the light emitted by the light source when viewed by the optical receiver after the light has propagated through the one or more optical fibers may have a wavelength that is influenced by fluid (e.g., blood 112) received by the blood detection layer. The optical receiver may generate optical data that is indicative of at least the wavelength of the light received by the optical receiver and transmit this optical data to the controller 300. In an instance in which the controller 300 comprises the optical receiver, any transmission at operation 404 may refer to an internal transmission.

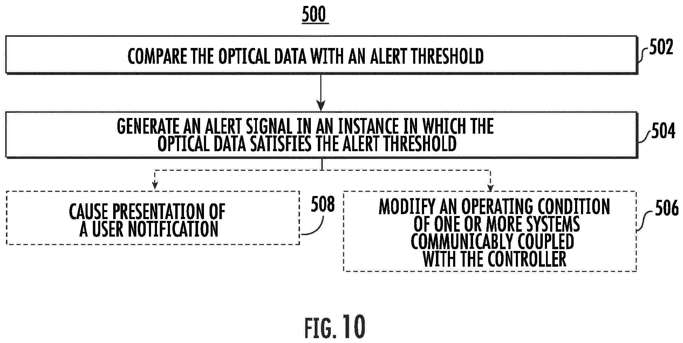

As shown in operation 406, the apparatus (e.g., controller 300) includes means, such as processor 302 or the like, for determining an amount of blood received by a blood detection layer based upon the optical data. As described above, a patient's blood may include one or more erythrocytes (e.g., red blood cells) that may be red in color (e.g., have a wavelength associated with red visible light). The optical fibers may contact this blood when blood is received by the blood detection layer such that the blood may impact or otherwise influence the wavelength of the light emitted by the light source, such that the wavelength of the light that is received by the optical receiver differs from the wavelength of the light emitted by the light source. Therefore, the wavelength of the light as indicated by the optical data may be analyzed (e.g., compared against one or more applicable thresholds) to determine the presence and/or the amount of blood received by the blood detection layer. For example, the optical receiver may generate optical data indicative of a wavelength of the light received by the optical receiver that is approximately 650 nm. The controller 300 may compare this optical data indictive of a wavelength of 650 nm with an applicable threshold (e.g., an alert threshold as described with reference to FIG. 10) to determine the presence of blood. In other words, the controller 300 may determine the presence of blood received by the blood detection layer 200 in an instance in which the optical data is indicative of a wavelength of light that is greater than approximately 620 nm but less than approximate 750 nm (e.g., 620 nm > 650 nm > 720 nm).

Furthermore, the amount determined at operation 406 may refer to a volume, density, and/or the presence of blood received by the sensing device 100. In other words, the controller 300 may be configured to, in some embodiments, detect any bleeding event associated with a patient (e.g., detect the presence of blood) regardless of the amount of blood associated with the bleeding event. For example, the controller 300 may determine optical data at operation 404 that satisfies an alert threshold as described above (e.g., optical data indicative of light having a wavelength associated with red visible light) and determine the presence of blood. In other embodiments, the controller 300 may be configured to determine an amount (e.g., volume, density, and/or the like) that is indicative of a hemorrhaging event as described hereafter with reference to FIG. 10. For example, the controller 300 may leverage, define, or otherwise employ one or more thresholds against which the optical data may be compared. In other words, optical data indicative of light having a wavelength closer to 720 nm may be indicative of a larger amount of blood than optical data indicative of light having a wavelength closer to 620 nm.

FIG. 10 illustrates a flowchart containing a series of operations for alert signal operations (e.g., method 500). The operations illustrated in FIG. 10 may, for example, be performed by, with the assistance of, and/or under the control of an apparatus (e.g., controller 300), as described above. In this regard, performance of the operations may invoke one or more of processor 302, memory 304, input/output circuitry 306, and/or communications circuitry 308.

As shown in operation 502, the apparatus (e.g., controller 300) includes means, such as processor 302, communications circuitry 308, or the like, for comparing the optical data with an alert threshold. As described above, in some embodiments, the controller 300 may employ one or more thresholds in order to quantify the amount and/or quantity of blood received by the blood detection layer and/or sensing device 100. By way of example, during a calibration procedure or the like, one or more blood detection layers (e.g., the blood detection layer 200 or the like) may be subjected to an amount of blood indicative of a hemorrhaging event, and the controller 300 may determine the wavelength of the light propagating through the blood detection layer in response to this amount of blood. Such a calibration procedure may be iteratively performed at various amounts of blood (e.g., based upon patient size, blood content, etc.) in order to determine an amount of blood that is indicative of a hemorrhaging event (e.g., the alert threshold). Said differently, the controller 300 may, for example, determine patient specific (e.g., based upon blood volume, blood content, or any other patient characteristic) thresholds against which the optical data may be compared at operation 502. In any embodiment, the controller 300 may leverage various machine learning techniques (e.g., supervised learning, unsupervised learning, neural networks, etc.) in order to improved generation of the alert thresholds described herein. Furthermore, although described herein with reference to a hemorrhaging event, the present disclosure contemplates that the alert threshold(s) described herein may be used to detect any event associated with a patient based upon the intended application of the sensing device 100.

Thereafter, as shown in operation 504, the apparatus (e.g., controller 300) includes means, such as processor 302, communications circuitry 308, or the like, for generating an alert signal in an instance in which the optical data satisfies the alert threshold. As described above, the optical data may be compared with one or more alert thresholds in order to determine the presence of blood received by the blood detection layer and/or the amount of blood received by the blood detection layer. For example, the controller 300 may iteratively emit light by the light source that propagates through the blood detection layer via the one or more optical fibers and is received by the optical receiver. The controller 300 may iteratively receive optical data indicative of the wavelength of the light that propagates through the blood detection layer. Given that the presence of blood impacts (e.g., changes) the wavelength of the light within the optical fiber(s), the comparison at operation 502 may be, for example, a comparison between the wavelength (e.g., defined by the optical data) and an alert threshold that includes a minimum wavelength (e.g., the lower bound of wavelengths associated with red visible light). Said differently, if the wavelength of the light within the optical fibers increases to a value that satisfies and/or exceeds the lower bound (e.g., approximately 620 nm) of the wavelength defined by the alert threshold, an alert signal may be generated.

In some embodiments, various alert thresholds may be used. For example, a first alert threshold may be used to detect the presence of blood received by the blood detection layer (e.g., a lower wavelength value). A subsequent or second alert threshold may be used to detect a hemorrhaging event or other hazardous condition (e.g. an increased wavelength or the upper bound (e.g. approximately 720 nm) of the wavelength associated with red visible light). In doing so, the sensing devices 100 of the present disclosure may operate to detect false positives associated with some blood contact that is prevalent in many surgeries, operations, etc. Said differently, such a first alert threshold may operate to prevent unnecessary resource diversion to a patient when blood is present, but the amount of blood isn't such that a hemorrhaging event is occurring. In this way, hospitals, clinics, etc. employing the sensing device 100 may operate to effectively prioritize emergency events.

In some embodiments, as shown in operation 506, the apparatus (e.g., controller 300) includes means, such as processor 302, communications circuitry 308, or the like, for modifying an operating condition of one or more systems (e.g., control systems 350) communicably coupled with the controller 300. In some embodiments, the controller 300 may be operably connected with various control systems 350 associated with one or more diagnostic systems, drug delivery systems, or the like for a particular patient. In some embodiments, the alert signal generated at operation 504 may include instructions configured to modify, augment, or otherwise change an operating condition of these control systems 350. For example, the sensing device 100 and/or the controller 300 may be operably connected with a drug delivery system (e.g., an example control system 350) that administers medication, such as anticoagulant or blood thinning agents. In response to detection of a hemorrhaging event as described above, the alert signal generated by the controller 300 may operate to halt administration of such medication by the example drug delivery system (e.g., an example control system 350). Although described herein with reference to anticoagulant administration, the present disclosure contemplates that the alert signal generated by the controller may be configured to cause any change associated with any control system 350 based upon the intended application of the sensing device 100.

In other embodiments, as shown in operation 508, the apparatus (e.g., controller 300) includes means, such as processor 302, communications circuitry 308, or the like, for causing presentation of a user notification. As described above, in some embodiments, the controller 300 may include one or more mechanisms for causing display of data generated by the controller 300. For example, the controller 300 may include or otherwise be operably coupled to a display screen configured to cause presentation of a user notification in response to the alert signal. By way of a particular example, the alert signal generated at operation 504 may include instructions for causing visual and/or audible presentation of a user notification that indicates that the optical data satisfies the alert threshold. In doing so, the user notification may indicate to a user that either blood is presently contacting the blood detection layer of the sensing device and/or that a hemorrhaging event or other emergency event is occurring.

FIGS. 9 and 10 thus illustrate flowcharts describing the operation of apparatuses, methods, and computer program products according to example embodiments contemplated herein. It will be understood that each flowchart block, and combinations of flowchart blocks, may be implemented by various means, such as hardware, firmware, processor, circuitry, and/or other devices associated with execution of software including one or more computer program instructions. For example, one or more of the operations described above may be implemented by an apparatus executing computer program instructions. In this regard, the computer program instructions may be stored by a memory 304 of the controller 300 and executed by a processor 302 of the controller 300. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture, the execution of which implements the functions specified in the flowchart blocks. The computer program instructions may also be loaded onto a computer or other programmable apparatus to cause a series of operations to be performed on the computer or other programmable apparatus to produce a computerimplemented process such that the instructions executed on the computer or other programmable apparatus provide operations for implementing the functions specified in the flowchart blocks.

The flowchart blocks support combinations of means for performing the specified functions and combinations of operations for performing the specified functions. It will be understood that one or more blocks of the flowcharts, and combinations of blocks in the flowcharts, can be implemented by special purpose hardware-based computer systems which perform the specified functions, or combinations of special purpose hardware with computer instructions.

## Claims

1. A sensing device for hemorrhage detection, the device comprising:
a blood detection layer comprising:
a permeable film; and
one or more optical fibers supported by the permeable film;
an optical sensor comprising:
a light source configured to emit light; and
an optical receiver in optical communication with the light source via the one or more optical fibers, the optical receiver configured to generate optical data responsive to the light emitted by the light source and received by the optical receiver via the one or more optical fibers, wherein the optical data is indicative of at least a wavelength of the light; and
a controller operably coupled with the optical sensor, wherein the controller is configured to:
receive the optical data generated by the optical receiver in response to the light emitted by the light source; and
determine an amount of blood received by the blood detection layer based upon the optical data.

2. The sensing device according to Claim 1, wherein the controller is further configured to:
compare the optical data with an alert threshold; and
generate an alert signal in an instance in which the optical data satisfies the alert threshold.

3. The sensing device according to Claim 2, wherein the alert signal is configured to cause presentation of a user notification.

4. The sensing device according to Claim 2, wherein the alert signal is configured to modify an operating condition of one or more systems communicably coupled with the controller.

5. The sensing device according to Claim 1, wherein the one or more optical fibers are supported by a first surface of the permeable film.

6. The sensing device according to Claim 5, further comprising an anti-penetration layer applied to a second surface of the permeable film, wherein the second surface is opposite the first surface.

7. The sensing device according to Claim 6, wherein the anti-penetration layer comprises a polyethylene (PE) or polypropylene (PP) microporous film configured to emit vapor to an external environment of the sensing device but preclude fluid transmission therethrough.

8. The sensing device according to Claim 5, further comprising a first diversion layer defining a first surface and a second surface opposite the first surface, wherein the second surface of the first diversion layer is applied to the first surface of the blood detection layer.

9. The sensing device according to Claim 8, further comprising an absorption layer defining a first surface and a second surface opposite the first surface, wherein the second surface of the absorption layer is applied to the first surface of the first diversion layer.

10. The sensing device according to Claim 9, further comprising a second diversion layer defining a first surface and a second surface opposite the first surface, wherein the second surface of the second diversion layer is applied to the first surface of the absorption layer.

11. The sensing device according to Claim 10, wherein the second diversion layer comprises an air through non-woven of polyethylene (PE) and polyethylene terephthalate (PET) bicomponent fibers or PE and polypropylene (PP) bicomponent fibers.

12. A method for hemorrhage detection, the method comprising:
emitting light from a light source to one or more optical fibers supported by a permeable film of a blood detection layer;
generating, by an optical receiver in optical communication with the light source via the one or more optical fibers, optical data responsive to the light emitted by the light source, wherein the optical data is indicative of at least a wavelength of the emitted light; and
determining an amount of blood received by a blood detection layer based upon the optical data.

13. The method according to Claim 12, wherein determining the amount of blood further comprises:
comparing the optical data with an alert threshold; and
generating an alert signal in an instance in which the optical data satisfies the alert threshold.

14. A computer program product for hemorrhage detection, the computer program product comprising at least one non-transitory computer-readable storage medium storing program instructions that, when executed, cause a system to:
emit light from a light source to one or more optical fibers supported by a permeable film of a blood detection layer;
generate, by an optical receiver in optical communication with the light source via the one or more optical fibers, optical data responsive to the light emitted by the light source, wherein the optical data is indicative of at least a wavelength of the emitted light; and
determine an amount of blood received by a blood detection layer based upon the optical data.

15. The computer program product according to Claim 14, the computer program product comprising at least one non-transitory computer-readable storage medium storing program instructions that, when executed, further cause the system to:
compare the optical data with an alert threshold; and
generate an alert signal in an instance in which the optical data satisfies the alert threshold.
